# EUROPÄISCHE PATENTSCHRIFT

(11) **EP 0 564 839 B1**
(45) Veröffentlichungstag und Bekanntmachung des Hinweises auf die Patenterteilung: **27.08.1997**
(21) Anmeldenummer: 93104009.1
(22) Anmeldetag: 12.03.1993
(51) Int. Cl.: G01N 33/52, G01N 31/22

(54) **Indikatorelement**
Indicator element
Elément indicateur

(30) Priorität: 27.03.1992 DE 9204126 U
(43) Veröffentlichungstag der Anmeldung: 13.10.1993
(73) Patentinhaber: MACHEREY, NAGEL & CO., D-52355 Düren (DE)
(72) Erfinder: Radmacher, Edmund, Dr., W-5160 Düren (DE)
(74) Vertreter: Paul, Dieter-Alfred, Dipl.-Ing.

(56) Entgegenhaltungen:
- FLUKA CHEMIKA-BIOCHEMIKA 1990/1991. Fluka Chemie AG. Buchs, Schweiz.

## Beschreibung

Die Erfindung betrifft ein Indikatorelement, insbesondere in Stäbchenform, mit einem Träger, der wenigstens ein einen Indikatorfarbstoff enthaltendes Indikatorfeld aufweist, wobei der Indikatorfarbstoff nicht ausblutend fixiert ist.

Zur schnellen und einfachen pH-Messung sind pH-Indikatorelemente - auch Indikatorpapiere genannt - seit langem bekannt und erfolgreich im Einsatz. Sie haben in der Regel eine flache Stäbchenform, die von einem Träger häufig aus saugfähigem Papier gebildet wird. Der Träger ist mit einem Indikatorfarbstoff versehen, beispielsweise durch Imprägnierung und anschließende Trocknung. Dabei können die Indikatorfarbstoffe einzeln oder in Gemischen vorliegen. Es können auch mehrere Indikatorfarbstoffe auf einem Träger aufgebracht sein, so daß verschiedene, voneinander getrennte Indikatorfelder gebildet werden. Damit lassen sich unterschiedlich große pH-Intervalle mit verschiedenen Skalenabständen abdecken.

Solche Indikatorstäbchen werden in die Prüflösung eingetaucht und nehmen dabei eine für den pH-Wert typische, reproduzierbare Färbung an. Mit Hilfe einer zugehörigen Farbskala, bei der in der Regel auf weißem Karton oder Papier im Druckverfahren eine Mehrzahl von Vergleichsfarben in Form von Vergleichsfeldern aufgedruckt sind, wird ein Farbvergleich vorgenommen. Das Indikatorfeld wird dann dem farblich passenden Vergleichsfeld zugeordnet, und es kann dann der neben oder auf dem Vergleichsfeld aufgedruckte pH-Wert als derjenige Wert, den die Prüflösung hat, abgelesen werden.

Bei Verwendung solcher Indikatorstäbchen besteht das Problem, daß der Indikatorfarbstoff leicht ausblutet und dann die Prüflösung, die oft noch benötigt wird oder wertvoll ist, verunreinigt. Dies erschwert auch den anschließenden Farbvergleich. Bei schwach gepufferten Lösungen ist es zudem notwendig, den Indikatorstreifen mehrere Minuten in die Prüflösung einzutauchen, da sich das pH-Gleichgewicht zwischen Prüflösung und Indikatorfarbstoff erst nach längerer Dauer einstellt. Dies führt - insbesondere im alkalischen Bereich - zu einem weitgehenden Ausbluten des Indikatorfarbstoffs und damit zur Unbrauchbarkeit der Meßmethode.

Schon vor langer Zeit sind deshalb Indikatorelemente entwickelt worden, auf deren Träger der Indikatorfarbstoff nicht ausblutend fixiert ist. Dies wird insbesondere durch drei bekannte Verfahren bewirkt. Bei dem ersten Verfahren kommen Indikatorfarben zum Einsatz, die aus einer chemischen Verbindung bestehen, in deren Moleküle Gruppen eingebaut sind, welche mit dem Träger eine chemische Verbindung eingehen (vgl. DE-AS 1 256 445; DE-AS 1 698 247). Bei dem zweiten Verfahren werden Indikatorfarbstoffe mit substantiven Eigenschaften, die mit dem Träger nicht kovalent verbunden werden, verwendet (DE-OS 24 36 257). Bei dem dritten Verfahren werden die Träger mit einem Indikatorfarbstoff imprägniert, wobei gleichzeitig oder anschließend ein farbfixierendes Hilfsmittel, beispielsweise eine organische Base mit nicht weniger als sechs Kohlenstoffatomen, zur Anwendung kommt (vgl. DE-AS 1 0̸30̸ 675).

Mit diesen Indikatorelementen konnte zwar das Problem des Ausblutens des Indikatorfarbstoffs gelöst werden. Die Indikatorstäbchen haben jedoch - genauso wie die eingangs beschriebenen, ausblutenden Indikatorfarbstoffe - noch den Nachteil, daß der Indikatorfarbstoff bei der Messung gefärbter oder trüber Prüflösung durch deren Eigenfarbe und Trübung infolge Farbaddition derart verändert wird, daß das Indikatorfeld einem falschen Vergleichsfeld auf der Farbskala zugeordnet wird oder eine Zuordnung sogar unmöglich ist. Entsprechend konnten Indikatorelemente mit nicht ausblutend fixierten Indikatorfarbstoffen bisher in gefärbten oder trüben Prüflösungen nicht oder nur begrenzt eingesetzt werden.

Bei Indikatorelementen mit ausblutenden Indikatorfarbstoffen hat man versucht, das vorgenannte Problem dadurch zu beseitigen, daß ein einziger Träger sowohl mit einem Indikatorfeld als auch mit mehreren Vergleichsfeldern versehen worden ist. Beim Eintauchen dieses Indikatorelements in die Prüflösung werden dann sowohl der Indikatorfarbstoff als auch die Vergleichsfarben durch die Eigenfarbe und/oder Trübung der Prüflösung in gleicher Weise verändert. Der Einfluß der Eigenfarbe und Trübung der Prüflösung wird also ausgeglichen (Kompensationsprinzip), so daß wieder eine eindeutige Zuordnung von Indikatorfeld zu einem der Vergleichsfelder möglich ist. Ungelöst ist jedoch bei diesen Indikatorpapieren bzw. -stäbchen nach wie vor das Problem des Ausblutens der Farben, was den Einsatzbereich aus den oben angegebenen Gründen stark einschränkt.

Der Erfindung liegt die Aufgabe zugrunde, ein Indikatorelement insbesondere in Stäbchenform derart auszubilden, daß es trotz Verwendung eines nicht ausblutenden Indikatorfarbstoffes zuverlässig auch in gefärbten oder trüben Prüflösungen eingesetzt werden kann.

Diese Aufgabe wird erfindungsgemäß dadurch gelöst, daß der Träger zusätzlich mit Vergleichsfarben versehene Vergleichsfelder aufweist, wobei die Vergleichsfarben ebenfalls nicht ausblutend fixiert sind. Nach der Erfindung wird also erstmals der Träger eines Indikatorelements, das einen nicht ausblutenden Indikatorfarbstoff aufweist, zusätzlich mit Vergleichsfeldern versehen. Da die Vergleichsfelder - wie das bzw. die Indikatorfeld(er) - nicht ausblutend fixiert sind, kann das Indikatorelement auch bei nach der Messung noch benötigten oder wertvollen Prüflösungen und insbesondere bei schwach gepufferten Lösungen zuverlässig eingesetzt werden, da die Prüflösung bei der Messung nicht beeinflußt wird und die Zuordnungsmöglichkeit von Indikatorfeld und passendem Vergleichsfeld eindeutig bleibt. Daneben ist aber auch der Einsatz in gefärbten und trüben Prüflösungen möglich, selbst wenn es dabei zu Farbadditionen kommt. Da die hierdurch bewirkten Farbänderungen beide Arten von Farben, also sowohl den Indikatorfarbstoff als auch die Vergleichsfarben, betrifft, tritt hier eine Kompensation ein mit der Folge, daß auch dann eine eindeutige Zuordnung von Indikatorfeld zu passendem Vergleichsfeld möglich ist. Damit ist ein Indikatorelement geschaffen, das sich durch bisher nicht erreichte Einsatzbreite und Zuverlässigkeit auszeichnet.

In Ausbildung der Erfindung ist vorgesehen, daß das Indikatorelement als dünnes und längliches Indikatorstäbchen mit zwei Flachseiten ausgebildet ist und daß das Indikatorfeld und die Vergleichsfelder auf einer der Flachseiten vorgeshen sind. Dabei sollten Indikatorfeld und Vergleichsfelder in Längsrichtung des Indikatorstäbchens hintereinander angeordnet sein, wobei das Indikatorfeld vorzugsweise im Mittenbereich zwischen zwei Vergleichsfeldern vorgesehen ist, um den Abstand zum am weitesten abliegenden Vergleichsfeld möglichst gering zu halten.

Für die Anbringung des Indikatorfarbstoffs und der Vergleichsfarben bestehen mehrere Möglichkeiten. So können der Indikatorfarbstoff und die Vergleichsfarben direkt auf den Träger aufgetragen und an diesem nicht ausblutend fixiert werden, beispielsweise durch Imprägnierung und anschließende Fixierung mit einem Fixiermittel. Abweichend davon besteht die Möglichkeit, den Indikatorfarbstoff und die Vergleichsfarben auf mit dem Träger verbundenen, separaten Trägerstreifen aufzutragen und an diesen nicht ausblutend zu fixieren. Dies eröffnet die Möglichkeit, für den Träger ein anderes Material als für die Trägerstreifen zu verwenden. Schließlich können aber der Indikatorfarbstoff und die Vergleichsfarben als am Träger befestigte Formkörper vorliegen, die aus einem entsprechenden Pulver hergestellt worden sind (vgl. DE-AS 1 698 247).

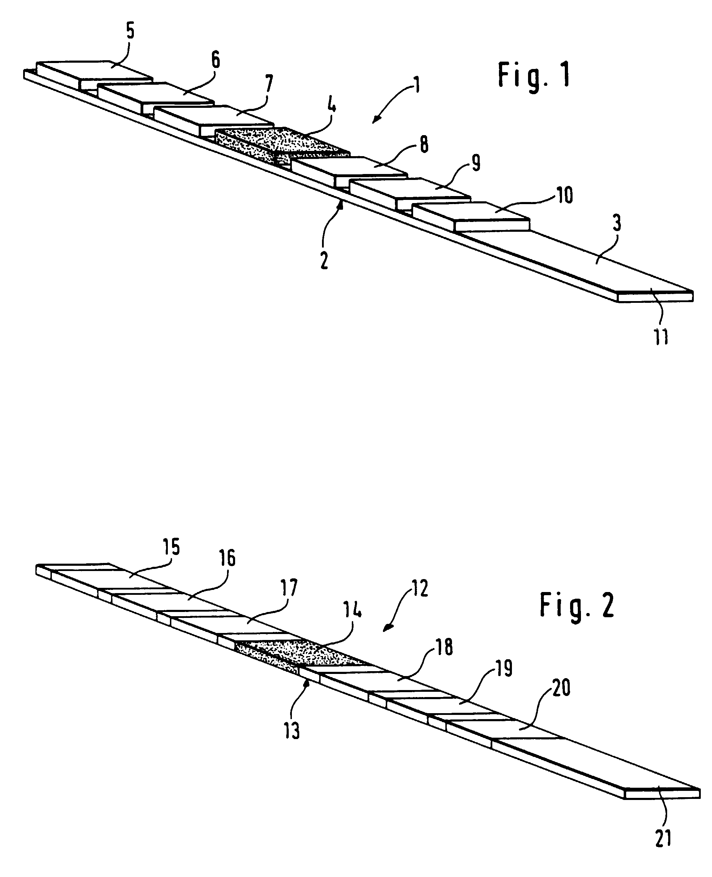

In der Zeichnung ist die Erfindung an Hand von zwei Ausführungsbeispielen näher veranschaulicht. Es zeigen:
- Figur (1): ein Indikatorstäbchen nach der Erfindung und
- Figur (2): ein anderes Indikatorstäbchen nach der Erfindung.

Das in Figur (1) dargestellte Indikatorstäbchen (1) hat einen länglichen dünnen Träger (2) mit einer obenseitigen Flachseite (3). Der Träger (2) kann aus Papier- oder Kunststoffmaterial bestehen.

An der Flachseite (3) des Trägers (2) sind insgesamt sieben Trägerstreifen befestigt. Von diesen Trägerstreifen bildet der mittlere ein Indikatorfeld (4), während die übrigen Trägerstreifen als Vergleichsfelder (5, 6, 7, 8, 9, 10) bestimmt sind. An dem das Indikatorfeld (4) bildenden Trägerstreifen ist ein Indikatorfarbstoff nicht ausblutend fixiert. An dem die Vergleichsfelder (5, 6, 7, 8, 9, 20) bildenden Trägerstreifen sind unterschiedliche Vergleichsfarben ebenfalls nicht ausblutend fixiert. Die Eigenschaft, in Prüflösungen nicht auszubluten, kann durch Verfahren erzielt werden, die in den eingangs zitierten Druckschriften im einzelnen dargestellt sind. Der Träger (2) hat ein freies Ende (11), an dem er beim Eintauchen in die Prüflösung mit den Fingern gehalten werden kann.

Das in Figur (2) dargestellte Indikatorstäbchen (12) weicht von dem in Figur (1) dargestellten Indikatorstäbchen (1) lediglich dadurch ab, daß Indikatorfarbstoff und Vergleichsfarben direkt auf dessen Träger (13) aufgetragen und durch eine entsprechende Nachbehandlung je nach Art des Farbstoffes nicht ausblutend fixiert worden ist. Das Indikatorstäbchen (12) hat in gleicher Anordnung wie der bei der Ausführung in Figur (1) ein mittiges Indikatorfeld (14) sowie sich nach beiden Seiten jeweils anschließende drei Vergleichsfelder (15, 16, 17, 18, 19, 20̸). Über das freie Ende (21) kann das Indikatorstäbchen (12) in eine Prüflösung eingetaucht werden, ohne daß die Finger der Bedienungsperson mit der Prüflösung in Berührung kommen.

Beim Eintauchen der Indikatorstäbchen (1, 12) in eine Prüflösung nehmen die Indikatorfelder (4, 14) auf Grund des darin gehaltenen Indikatorfarbstoffs eine dem jeweiligen pH-Wert der Prüflösung entsprechende Farbe an. Dabei tritt kein Ausbluten des Indikatorfarbstoffs und der Vergleichsfarben ein. Soweit die Prüflösung eine Eigenfarbe oder eine Trübung aufweist, werden hierdurch sowohl der Indikatorfarbstoff als auch die Vergleichsfarben durch Farbadditionen in gleicher Weise beeinflußt. Nach der Entnahme der Indikatorstäbchen (1, 12) können die Indikatorfelder (4, 14) einem Vergleichsfeld (5, 6, 7, 8, 9, 10̸) bzw. (15, 16, 17, 18, 19, 20̸) mit gleicher oder ähnlicher Farbe zugeordnet werden. Wenn die Vergleichsfelder (5, 6, 7, 8, 9, 10̸) bwz. (15, 16, 17, 18, 19, 20̸) mit dem der Farbe entsprechenden pH-Wert versehen sind, beispielsweise durch Aufdruck, kann der pH-Wert der Prüflösung sofort abgelesen werden.

## Patentansprüche

1. Indikatorelement, insbesondere in Stäbchenform, mit einem Träger, der wenigstens ein einen Indikatorfarbstoff enthaltendes Indikatorfeld aufweist, wobei der Indikatorfarbstoff nicht ausblutend fixiert ist,
dadurch gekennzeichnet, daß der Träger (2, 13) zusätzlich mit Vergleichsfarben versehene Vergleichsfelder (5 bis 10̸) bzw. (15 bis 20̸) aufweist, wobei die Vergleichsfarben ebenfalls nicht ausblutend fixiert sind.

2. Indikatorelement nach Anspruch (1),
dadurch gekennzeichnet, daß das Indikatorelement als dünnes und längliches Indikatorstäbchen (1, 12) mit zwei Flachseiten (3) ausgebildet ist und daß das bzw. die Indikatorfeld(er) (4, 14) und die Vergleichsfelder (5 bis 10̸) bzw. (15 bis 20̸) auf einer der Flachseiten (3) vorgesehen sind.

3. Indikatorelement nach Anspruch (2),
dadurch gekennzeichnet, daß Indikatorfeld (4, 14) und Vergleichsfelder (5 bis 10̸) bzw. (15 bis 20̸) in Längsrichtung des Indikatorstäbchens (1, 12) hintereinander angeordnet sind, wobei das Indikatorfeld (4, 14) im Mittenbereich zwischen zwei Vergleichsfeldern (7, 8) bzw. (17, 18) vorgesehen ist.

4. Indikatorelement nach einem der Ansprüche (1) bis (3),
dadurch gekennzeichnet, daß der Indikatorfarbstoff und die Vergleichsfarben direkt auf dem Träger (13) aufgetragen und an diesem nicht ausblutend fixiert sind.

5. Indikatorelement nach einem der Ansprüche (1) bis (3),
dadurch gekennzeichnet, daß der Indikatorfarbstoff und die Vergleichsfarben auf mit dem Träger (2) verbundenen, separaten Trägerstreifen aufgetragen und an diesen nicht ausblutend fixiert sind.

## Claims

1. An indicator element, particularly in the form of a small stick, having a support which comprises at least one indicator area containing an indicator dye, wherein the indicator dye is fixed so that it does not leach out, characterised in that the support (2, 13) additionally comprises comparison areas (5 to 10) or (15 to 20) provided with comparison colours, wherein the comparison colours are likewise fixed so that they do not leach out.

2. An indicator element according to claim (1),
characterised in that the indicator is formed as a thin, elongated small indicator stick (1, 12) with two flat faces (3), and that the indicator area or areas (4, 14) and the comparison areas (5 to 10) or (15 to 20) are provided on one of the flat faces (3).

3. An indicator element according to claim (2).
characterised in that the indicator area (4, 14) and the comparison areas (5 to 10) or (15 to 20) are disposed in succession in the longitudinal direction of the small indicator stick (1, 12), wherein the indicator area (4, 14) is provided in the middle region between two comparison areas (7, 8) or (17, 18).

4. An indicator element according to any one of claims (1) to (3),
characterised in that the indicator dye and the comparison colours are directly deposited on the support (13) and are fixed thereto so that they do not leach out.

5. An indicator element according to any one of claims (1) to (3),
characterised in that the indicator dye and the comparison colours are deposited on separate support strips joined to the support (2) and are fixed to the support strips so that they do not leach out.

## Revendications

1. Elément indicateur revêtant notamment la forme d'un bâtonnet et présentant un substrat muni d'au moins une zone indicatrice renfermant un colorant indicateur, le colorant indicateur étant fixé de manière non migrante,
caractérisé par le fait que le substrat (2, 13) comporte, additionnellement, des zones comparatives (5 à 10, respectivement 15 à 20) munies de matières colorantes comparatives, les matières colorantes comparatives étant semblablement fixées de manière non migrante.

2. Elément indicateur selon la revendication 1,
caractérisé par le fait que l'élément indicateur est réalisé sous la forme d'un bâtonnet indicateur (1, 12) mince et allongé, comprenant deux faces aplaties (3) ; et par le fait que la ou les zone(s) indicatrice(s) (4, 14) et les zones comparatives (5 à 10, respectivement 15 à 20) sont prévues sur l'une des faces aplaties (3).

3. Elément indicateur selon la revendication 2,
caractérisé par le fait que la zone indicatrice (4, 14) et les zones comparatives (5 à 10, respectivement 15 à 20) sont agencées en succession dans le sens longitudinal du bâtonnet indicateur (1, 12), la zone indicatrice (4, 14) étant prévue dans la région médiane située entre deux zones comparatives (7, 8, respectivement 17, 18).

4. Elément indicateur selon l'une des revendications 1 à 3,
caractérisé par le fait que le colorant indicateur et les matières colorantes comparatives sont directement déposés sur le substrat (13), et fixés à ce dernier de manière non migrante.

5. Elément indicateur selon l'une des revendications 1 à 3,
caractérisé par le fait que le colorant indicateur et les matières colorantes comparatives sont déposés sur des bandes-substrats distinctes, reliées au substrat (2), et sont fixés à ces dernières de manière non migrante.
